# EUROPÄISCHE PATENTSCHRIFT

(11) **EP 0 441 997 B1**
(45) Veröffentlichungstag und Bekanntmachung des Hinweises auf die Patenterteilung: **27.12.1995**
(21) Anmeldenummer: 90102737.5
(22) Anmeldetag: 12.02.1990
(51) Int. Cl.: A61B 17/225, G10K 15/04

(54) **Medizinischer Ultraschall-Applikator zur Verwendung in einem von akustischen Stosswellen durchlaufenen Ausbreitungsmedium**
Medical ultrasonic applicator for use in an acoustic shock-wave-conducting propagation-medium
Applicateur médical ultrasonore pour utilisation dans un milieu de propagation acoustique transmettant des ondes de choc

(43) Veröffentlichungstag der Anmeldung: 21.08.1991
(73) Patentinhaber: SIEMENS AKTIENGESELLSCHAFT, 80333 München (DE)
(72) Erfinder: Harder, Rudolf, Dipl.-Ing.(FH), D 8550 Forchheim (DE); Hetzel, Gert, Dipl.-Ing., D-8520 Erlangen (DE); Kaarmann, Hans, Dipl.-Phys.Dr., D-8520 Buckenhof (DE); Köhler, Georg, Dipl.-Ing., D-8618 Geisfeld (DE); Kühnke, Hermann, Dipl.Ing.(FH), D-8521 Münchaurach (DE); Rohwedder, Arnim, Dipl.-Ing., D-8520 Erlangen (DE); Schätzle, Ulrich, Dipl.-Ing., D-8524 Neunkirchen (DE)

(56) Entgegenhaltungen:
- EP-A- 0 133 665
- EP-A- 0 301 360
- EP-A- 0 434 936
- DE-A- 3 608 877
- FR-A- 2 587 493
- US-A- 4 620 545
- US-A- 4 622 969

## Beschreibung

Die Erfindung betrifft einen Stoßwellengenerator zur Erzeugung von Stoßwellen in einem akustischen Ausbreitungsmedium mit einem Ultraschall-Applikator, mittels dessen für Ortungszwecke Ultraschallwellen in das akustische Ausbreitungsmedium einleitbar sind und der wenigstens einen Ultraschall-Transducer aufweist, der in einem Ghäuse mit einem Schallaustrittsfenster für die mittels des Ultraschall-Transducers erzeugten Ultraschallwellen aufgenommen ist. Die Erfindung betrifft außerdem einen Ultraschall-Applikator zur Verwendung in einem von akustischen Stoßwellen durchlaufenen Ausbreitungsmedium, welcher ein Gehäuse und wenigstens einen innerhalb des Gehäuses angeordneten Ultraschall-Iransducer aufweist, wobei das Gehäuse ein Schallaustrittsfenster für die mittels des Ultraschall-Transducers erzeugten Ultraschallwellen aufweist.

Ein Stoßwellengenerator der eingangs genannten Art ist in der EP-A-0 301 360 beschrieben. Dieser Stoßwellengenerator, der zum nicht invasiven Zertrümmern von Konkrementen im Körper eines Lebewesens dient, weist ein Gehäuse auf, in dem eine Stoßwellenquelle aufgenommen ist. Diese dient dazu, in die in dem an seinem Applikationsende mittels eines flexiblem Sackes verschlossenen Gehäuse als Ausbreitungsmedium vorhandene Flüssigkeit Stoßwellen einzuleiten, die mittels einer akustischen Linse fokussiert werden. Um den Stoßwellengenerator relativ zu dem Körper eines zu behandelnden Lebewesens so ausrichten zu können, daß sich ein zu zertrümmerndes Konkrement in dem Fokus der Stoßwellen befindet, weist der bekannte Stoßwellengenerator einen zu einem Ultraschall-Ortungssystem gehörigen Ultraschall-Applikator auf, der dazu dient, ein Ultraschallbild des den Fokus umgebenden Bereiches des Körpers des Lebewesens zu erzeugen. Der in einer zentralen Bohrung des Stoßwellengenerators unter anderem längsverschieblich aufgenommene Ultraschall-Applikator muß, um einwandfreie Ultraschallbilder erzeugen zu können, unter Zwischenfügung der flexiblen Membran an die Körperoberfläche des zu behandelnden Lebewesens angepreßt werden. In dieser Position wird der Ultraschall-Applikator, obwohl infolge der zur Aufnahme des Ultraschall-Applikators vorgesehenen zentralen Bohrung an sich ein stoßwellenfreier Bereich vorliegt, dennoch von Anteilen der vorbeilaufenden Stoßwellen getroffen, so daß die Gefahr besteht, daß Teile im Inneren des Ultraschall-Applikators Schaden nehmen. Außerdem besteht die Gefahr, daß das den Ultraschall-Transducer umgebende Gehäuse durch Kavitationswirkung in einer solchen Weise beschädigt wird, daß Wasser in den an sich ölgefüllten Innenraum des Gehäuses des Ultraschall-Applikators eindringen kann, was zum Ausfall des Ultraschall-Applikators führt.

Ein ähnlicher Stoßwellengenerator ist aus dem DE-GM 88 09 253 bekannt. Auch hier ist zur Fokussierung der mittels der Stoßwellenquelle erzeugten Stoßwellen eine akustische Linse vorgesehen. Um deren Abbildungsfehler zu korrigieren, ist in dem Ausbreitungsweg der Stoßwellen ein hohlkegeliger Ablenkkörper angeordnet, der im Bereich seiner Mantelfläche aus Messing oder Stahl besteht. Als Ausbreitungsmedium für die Stoßwellen ist Wasser vorgesehen. Im Inneren des hohlkegeligen Körpers ist der Ultraschall-Applikator einer Ultraschall-Ortungseinrichtung angeordnet. Dabei soll der hohlkegelige Körper den Ultraschall-Applikator gegen die Einwirkung der Stoßwellen schützen. Da die Grundfrequenz der Stoßwellen gewöhnlich bei 0,5 MHz liegt und eine nennenswerte Schalldämmwirkung erst dann erzielt werden kann, wenn die Wandstärke des hohlkegeligen Körpers wenigstens gleich einer Wellenlänge der Grundschwingung einer sich in dem Werkstoff des hohlkegeligen Körpers ausbreitenden Stoßwelle ist, wäre bei Messing als Werkstoff für den hohlkegeligen Körper eine Wandstärke von wenigstens 9 mm erforderlich. Hierbei ist eine Schallausbreitungsgeschwindigkeit in Messing von ca. 4500 m/s zugrunde gelegt. Derartige Wandstärken des hohlkegeligen Körpers sind in der Praxis schon aus Platzgründen nicht realisierbar. Um den Ultraschall-Applikator vor den die Wandung des hohlkegeligen Körpers durchdringenden Anteilen der Stoßwellen zu schützen, ist daher vorgesehen, daß der hohlkegelige Körper in seinem Inneren aus einem Kunststoffschaum besteht, in dem die genannten Anteile der Stoßwellen absorbiert werden. Ein wirksamer Schutz des Ultraschall-Applikators ist im Falle der beschriebenen Lösung also nur möglich, wenn zum einen der hohlkegelige Körper eine ausreichende Wandstärke aufweist und zum anderen eine ausreichend starke Schicht des Kunststoffschaumes zwischen dem Gehäuse des Ultraschall-Applikators und der Innenwand des hohlkegeligen Körpers vorhanden ist. Mit der bekannten Lösung ist also ein hoher Bauraumbedarf verbunden. Außerdem müssen spezielle Maßnahmen zur Halterung des hohlkegeligen Körpers getroffen werden, was den konstruktiven Aufwand erhöht.

Aus der DE-A-36 08 877 ist ein Stoßwellengenerator gemäß Oberbegriff des Anspruchs 1 mit einem für Ortungszwecke vorgesehenen Ultraschall-Transducer als Teil eines Ultraschall-Applikators gemäß Oberbegriff des Anspruchs 5 bekannt. An dem Ultraschall-Transducer ist ein kegeliger Körper angesetzt. Letzterer ist außen mit einer Schicht aus einem Stoßwellen absorbierendem Material mit einer gewellten Oberfläche versehen. Unter dieser Schicht befindet sich eine Schicht aus einem Stoßwellen reflektierenden Material. Der Innenraum des durch den Ultraschall-Transducer abgeschlossenen kegeligen Körpers ist mit Luft gefüllt, die gegen die Transmission von Stoßwellen isolieren soll.

In der prioritätsälteren, aber nicht vorveröffentlichten EP-A-0 434 936 ist ein Stoßwellengenerator mit einem für Ortungszwecke vorgesehenen Ultraschall-Transducer beschrieben, der in einem Gehäuse aufgenommen ist, dem in einem Bereich eine Dämpfungsschicht vorgelagert ist, die aus mit gashaltigen Füllstoffen versehenem oder geschäumtem Kunststoff, Gummi oder Silikonkautschuk oder aus einem Gemisch aus Zwei-Komponentensilikon und micropheres (Hohlglaskugeln) besteht.

Der Erfindung liegt die Aufgabe zugrunde, einen Stoßwellengenerator der eingangs genannten Art so auszubilden, daß auf einfache, kostengünstige und bauraumsparende Weise ein Schutz des Ultraschall-Applikators vor der Einwirkung der Stoßwellen gewährleistet ist. Der Erfindung liegt außerdem die Aufgabe zugrunde, einen Ultraschall-Applikator so auszubilden, daß er ohne Gefahr von Beschädigungen in einem von akustischen Stoßwellen durchlaufenen Ausbreitungsmedium eingesetzt werden kann.

Der den Stoßwellengenerator betreffende Teil der Aufgabe wird nach der Erfindung gemäß Anspruch 1 dadurch gelöst, daß wenigstens dem im Ausbreitungsweg der Stoßwellen befindlichen, an das Schallaustrittsfenster im wesentlichen angrenzenden Bereich des Gehäuses ein einziger Stoff vorgelagert ist, dessen akustische Impedanz erheblich von der des akustischen Ausbreitungsmediums abweicht, wobei die Schallausbreitungsgeschwindigkeit in dem Stoff erheblich geringer als in dem akustischen Ausbreitungsmedium ist. Infolge der von der des akustischen Ausbreitungsmediums erheblich abweichenden akustischen Impedanz des Stoffes, unter einer erheblichen Abweichung soll hier eine Abweichung in der Größenordnung von wenigstens einer Zehnerpotenz verstanden werden, werden diejenigen Anteile der Stoßwellen, die den Ultraschall-Applikator gefährden könnten, an den Grenzflächen des Stoffes reflektiert, wobei die nicht reflektierten Anteile um so geringer sind, je stärker die akustische Impedanz des Stoffes von der des Ausbreitungsmediums abweicht. Die Anwesenheit des Stoffes kann sich auf diejenigen Bereiche beschränken, die sich im Ausbreitungsweg derjenigen Anteile der Stoßwellen befinden, die ohne die Anwesenheit des Stoffes zu den gefährdeten Teilen des Ultraschall-Applikators gelangen könnten. Dabei ist zu beachten, daß als Ausbreitungsweg nicht nur der direkte Weg von der Stoßwellenquelle zu den gefährdeten Teilen in Frage kommt, sondern daß schädliche Anteile von Stoßwellen auch durch Beugung oder Reflexion zu den gefährdeten Teilen gelangen können. Bezüglich nicht reflektierter Anteile der Stoßwellen wird bei geringem Beuraumbedarf auch eine Schalldämmung erreicht, da die Schallausbreitungsgeschwindigkeit in dem Stoff erheblich geringer als in dem akustischen Ausbreitungsmedium ist. Es kann dann bereits mit einer sehr geringen Schichtdicke eine sehr hohe Schalldämmungswirkung erzielt werden. Eine wirksame Schalldämmung bezüglich der nicht reflektierten Stoßwellenanteile wird dabei erreicht, wenn der Stoff gemäß einer bevorzugten Variante in einer Schichtdicke vorhanden ist, die wenigstens gleich einer Wellenlänge der Grundschwingung einer sich in dem Stoff ausbreitenden Stoßwelle ist. Gemäß einer Ausführungsform der Erfindung handelt es sich bei dem Stoff um ein gasförmiges Medium, insbesondere Luft. Es hat sich gezeigt, daß bei flüssigen akustischen Ausbreitungsmedien bereits ein Luft- oder Gasvolumen mit einer in Ausbreitungsrichtung der Stoßwellen gemessenen Schichtdicke von wenigen Millimetern zu einer Verringerung der im Bereich des Ultraschall-Transducers vorliegenden Stoßwellen-Schalleistung in der Größenordnung einer Zehnerpotenz bewirkt. Es wird also deutlich, daß durch die Erfindung auf einfache, kostengünstige und bauraumsparende Weise ein Schutz des Ultraschall-Applikators vor der Stoßwelleneinwirkung erreicht wird. Unter einer gegenüber der im akustischen Ausbreitungsmedium erheblich geringeren Schallgeschwindigkeit soll übrigens eine Schallgeschwindigkeit verstanden werden, die höchstens gleich einem Drittel der Schallausbreitungsgeschwindigkeit in dem Ausbreitungsmedium ist.

Um den Stoff, insbesondere dann, wenn es sich um ein gasförmiges Medium handelt, an Ort und Stelle halten zu können, ist vorgesehen, daß der Stoff in den Poren eines geschlossenporigen Schaumes oder in einem Schlauch, der mehrere Windungen aufweisen kann, oder in in einem Trägermaterial eingebetteten Hohlkörpern oder in einer einen Hohlraum umschließenden Kapselung eingeschlossen ist.

Falls das Gehäuse einen etwa rohrförmigen Abschnitt aufweist, der durch das Schallaustrittsfenster abgeschlossen ist, ist gemäß einer Ausführungsform der Erfindung vorgesehen, daß wenigstens der an das Schallaustrittsfenster anschließende etwa rohrförmige Bereich von dem Stoff ringförmig umgeben ist.

Der den Ultraschall-Applikator petreffende Teil der Aufgabe wird nach der Erfindung gemäß Anspruch 5 dadurch gelöst, daß wenigstens dem an das Schallaustrittsfenster im wesentlichen angrenzenden Bereich des Gehäuses ein einziger Stoff vorgelagert ist, dessen akustische Impedanz von der von der des von Stoßwellen durchlaufenen akustischen Ausbreitungsmediums erheblich abweicht, wobei die Schallausbreitungsgeschwindigkeit in dem Stoff erheblich geringer als im Ausbreitungsmedium ist. Da bei medizinischen Anwendungen als Ausbreitungsmedium in der Regel eine Substanz vorgesehen ist, deren akustische Impedanz der von Körpergewebe möglichst genau entspricht, weicht also die akustische Impedanz des dem Gehäuse vorgelagerten Stoffes von der des akustischen Ausbreitungsmediums ganz erheblich ab, mit der Folge, daß in der bereits im Zusammenhang mit dem Stoßwellengenerator beschriebenen Weise eine Reflexion der Stoßwellen an Grenzflächen des Stoffes erfolgt. Gemäß bevorzugten Varianten der Erfindung ist auch im Falle des Ultraschall-Applikators vorgesehen, daß der Stoff in einer Schichtdicke vorhanden ist, die wenigstens gleich einer Wellenlänge der Grundschwingung einer sich in dem Stoff ausbreitenden Stoßwelle ist, bzw. daß die Schallausbreitungsgeschwindigkeit in dem Stoff erheblich geringer als in Körpergewebe ist.

Ausführungsbeispiele der Erfindung sind in den beigefügten Zeichnungen dargestellt. Es zeigen:
- Fig. 1: in schematischer Darstellung einen Längsschnitt durch einen in einem erfindungsgemäßen Ultraschall-Applikator enthaltenden erfindungsgemäßen Stoßwellengenerator,
- Fig. 2: in vergrößerter Darstellung einen Längsschnitt durch ein Detail des Stoßwellengenerators bzw. des Ultraschall-Applikators gemäß Fig. 1, und
- Fig. 3 bis 5: Varianten des Details gemäß Fig. 2.

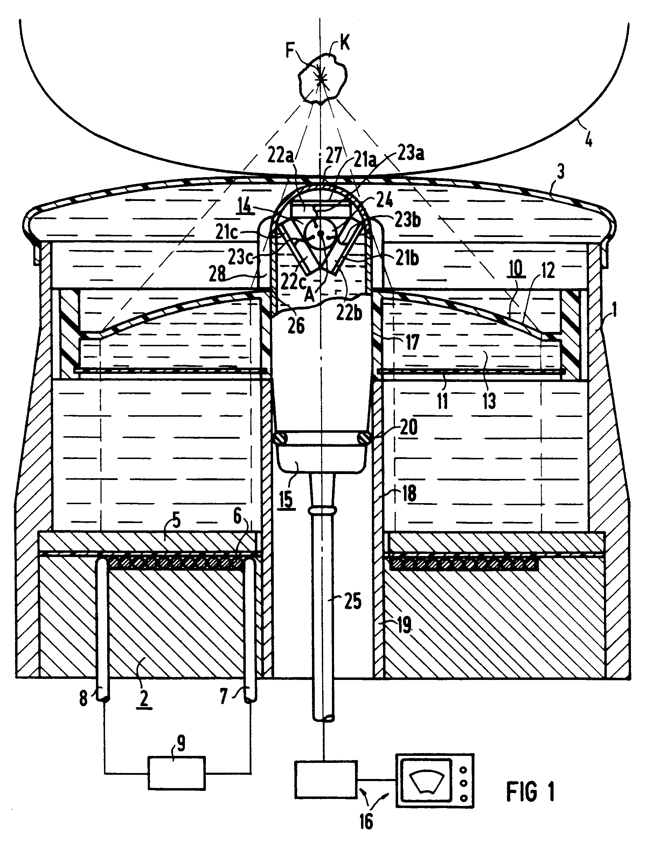

Der in Fig. 1 dargestellte erfindungsgemäße Stoßwellengenerator dient dazu, ein in einem Lebewesen befindliches Konkrement K, z.B. einen Nierenstein, berührungslos zu zertrümmern und weist ein etwa rohrförmiges Gehäuse 1 auf, an dessen einem Ende eine insgesamt mit 2 bezeichnete Stoßwellenquelle vorgesehen ist. An seinem anderen Ende ist das Gehäuse 1 mittels eines flexiblen Balges 3 verschlossen, der dazu dient, den Stoßwellengenerator zur akustischen Kopplung an den im Querschnitt angedeuteten Körper 4 eines zu behandelnden Lebewesens anpressen zu können. Der Innenraum des Gehäuses 1 ist mit Wasser gefüllt, das als akustisches Ausbreitungsmedium für die von der Stoßwellenquelle 2 ausgehenden Stoßwellen vorgesehen ist.

Bei der Stoßwellenquelle 2 handelt es sich um eine elektromagnetische Stoßwellenquelle, wie sie in der DE-OS 33 28 051 näher beschrieben ist. Die Stoßwellenquelle 2 weist eine ebene kreisringförmige Membran 5 auf, die mit ihrer einen Seite an das in dem Gehäuse 1 befindliche Wasser angrenzt. Der anderen Seite der aus einem elektrisch leitenden Werkstoff gebildeten Membran 5 gegenüberliegend ist eine Flächenspule 6 mit spiralförmig angeordneten Windungen vorgesehen, die über Anschlüsse 7, 8 mit einer schematisch angedeuteten Generatoreinrichtung 9 verbunden ist, mittels derer sie mit Hochspannungsimpulsen beaufschlagbar ist. Wird die Flächenspule 6 mit einem Hochspannungsimpuls beaufschlagt, bewegt sich die Membran 5 von der Flächenspule 6 schlagartig weg. Infolge dieser Bewegung wird in das Wasser ein im wesentlicher ebener Druckimpuls eingeleitet, der sich auf seinem Weg durch das Wasser zu einer Stoßwelle aufsteilt. Im folgenden wird der Einfachheit halber stets der Begriff Stoßwelle verwendet werden. Die Ausbreitungsrichtung der Stoßwellen entspricht der Richtung der Mittelachse der Stoßwellenquelle.

Um die erzeugten ebenen Stoßwellen in der zur Zertrümmerung von Konkrementen erforderlichen Weise fokussieren zu können, ist im Wasser innerhalb des Gehäuses 1 eine insgesamt mit 10 bezeichnete akustische Sammellinse zwischen der Stoßwellenquelle 2 und dem Balg 3 angeordnet. Die akustische Sammellinse 10 ist als Flüssigkeitslinse ausgeführt. Sie besitzt eine aus Polymethylpentene (TPX) gebildete Eintrittswand 11, eine aus Teflon (eingetragenes Warenzeichen) gebildete Austrittswand 12 und als zwischen Eintritts- und Austrittswand 11 und 12 eingeschlossene Linsenflüssigkeit 13 eine Fluor-Kohlenstoff-Flüssigkeit, z.B. Flutec PP3 oder Fluorinert FC 75 (eingetragene Warenzeichen). Da in den genannten Linsenflüssigkeiten die Schallausbreitungsgeschwindigkeit geringer als in Wasser ist, bewirkt die plankonvexe Gestalt der Sammellinse 10 eine Fokussierung der zunächst ebenen Stoßwellen auf eine mit F bezeichnete Fokuszone, die auf der Mittelachse der Stoßwellenquelle 2 liegt. Um den Stoßwellengenerator und den Körper 4 des zu behandelnden Lebewesens relativ zueinander so ausrichten zu können, daß ein im Körper 4 des zu behandelnden Lebewesens befindliches Konkrement K sich wie in der Fig. 1 dargestellt im Fokus F der Stoßwellen befindet, ist ein Ultraschall-Applikator 15 vorhanden, der in an sich bekannter Weise als mechanischer Sektor-Scanner ausgebildet ist und es im Zusammenwirken mit einer nur schematisch angedeuteten elektronischen Einrichtung 16 gestattet, Ultraschall-B-Bilder einer die Mittelachse der Stoßwellenquelle und den Fokus F der Stoßwellen enthaltenden Schicht des Körpers 4 zu erzeugen.

Der Ultraschall-Applikator 15 ist in seiner in Fig. 1 gezeigten Stellung in einer zentralen Bohrung 17 der Sammellinse 10 aufgenommen, wobei sich die Bohrung in einem Rohr 18 fortsetzt, das sich durch eine zentrale Bohrung 19 der Stoßwellenquelle 2 erstreckt. Das Rohr 18 ist sowohl mit der Sammellinse 10 als auch mit der Stoßwellenquelle 2 flüssigkeitsdicht verbunden. Der Ultraschall-Applikator 15 ist in der Bohrung 17 und dem Rohr 18 längsverschieblich und um die Mittelachse der Stoßwellenquelle 2 drehbar aufgenommen, wobei die entsprechenden Verstellmittel nicht dargestellt sind. Dabei ist mittels geeigneter, schematisch angedeuteter Dichtmittel 20 sichergestellt, daß der Ultraschall-Applikator 15 in der Bohrung 17 bzw. dem Rohr 18 flüssigkeitsdicht aufgenommen ist.

Wie aus der teilweise aufgebrochenen Darstellung des Ultraschall-Applikators 15 ersichtlich ist, enthält dieser drei Ultraschall-Transducer 21a, 21b, 21c, die jeweils mit einem Backing 22a, 22b, 22c verklebt sind. Die Ultraschall-Transducer 21a, 21b, 21c sind mittels ihrer Backings 22a, 22b, 22c an einem Rotor 14 befestigt. Dieser ist in nicht dargestellter Weise mittels eines Motors, erforderlichenfalls unter Zwischenschaltung geeigneter Getriebe, zur Rotation um eine rechtwinklig zur Mittelachse der Stoßwellenquelle 2 verlaufende Achse A antreibbar. Die zur Bilderzeugung erforderlichen Signale werden zwischen den Ultraschall-Transducern 21a, 21b, 21c und der elektronischen Einrichtung 16 und umgekehrt über schematisch angedeutete Leitungen 23a, 23b, 23c übertragen, mittels derer die Ultraschall-Transducer 21a, 21b, 21c über einen nur schematisch angedeuteten Schleifscheibenkontakt 24 mit dem zu der elektronischen Einrichtung 16 führenden Kabel 25 verbunden sind.

Der Ultraschall-Applikator 15 weist ein etwa hohlzylindrisches, rohrförmiges, die Ultraschall-Transducer 21a, 21b, 21c aufnehmendes Gehäuse 26 auf, das an seinem dem Fokus F zugewandten Ende ein koppelförmiges Verschlußteil aufweist, dessen dem Fokus F benachbarter Bereich als Schallaustrittsfenster 27 für die mittels des Ultraschall-Applikators 15 erzeugten Ultraschallwellen dient. Der die Ultraschall-Transducer 21a, 21b, 21c aufnehmende Raum des Gehäuses 26 ist mit einer Flüssigkeit, z.B. einem geeigneten Öl, gefüllt.

Wie anhand der in Fig. 1 strichliert eingetragenen Randstrahlen einer Stoßwelle erkennbar ist, wird der Ultraschall-Applikator 15, wenn er in der für eine gute Bildqualität erforderlichen Weise unter Zwischenschaltung des Balges 3 wie in Fig. 1 dargestellt an der Oberfläche des Körpers 4 anliegt, im Bereich der Ultraschall-Transducer 21a, 21b, 21c von den Stoßwellen gestreift, wobei Anteile der Stoßwellen in den flüssigkeitsgefüllten Innenraum innerhalb des Ultraschall-Applikators 15 gelangen können. Dabei besteht bei Stoßwellengeneratoren bzw. Ultraschall-Applikatoren nach dem Stand der Technik die Gefahr, daß Teile im Inneren des Ultraschall-Applikators 15 Schaden nehmen. Außerdem besteht die Gefahr, daß unter Stoßwelleneinwirkung das Gehäuse 26 infolge von Kavitationserscheinungen lokale Zerstörungen erfahren, die ein Eindringen von Wasser in das Innere des Ultraschall-Applikators 15 ermöglichen.

Um diese Schäden, die den Ausfall des Ultraschall-Applikators bzw. des Stoßwellengenerators nach sich ziehen, zu vermeiden, ist im Falle der Erfindung dem im Ausbreitungsweg der Stoßwellen befindlichen Bereich des Gehäuses 26 des Ultraschall-Applikators 5 ein Luftvolumen vorgelagert, das im Inneren einer den Ultraschall-Applikator 15 ringförmig umgebenden Kapselung 28 eingeschlossen ist. Die Kapselung 28 grenzt mit ihrem einen Ende etwa an das Schallaustrittsfenster 27 an und erstreckt sich über eine solche Länge entlang des rohrförmigen Gehäuses 26, daß sich das in ihr eingeschlossene Luftvolumen im Ausbreitungsweg derjenigen Anteile der Stoßwellen befindet, die zu den Ultraschall-Transducern 21a, 21b, 21c gelangen könnten. Es versteht sich, daß sich das Luftvolumen nicht vor den als Schallaustrittsfenster 27 dienenden Bereich des Verschlußteiles erstreckt, da dies zu einer Beeinträchtigung der Ausbreitung der mittels des Ultraschall-Applikators 15 erzeugten Ultraschallwellen führen würde. Die Kapselung 28 liegt spaltlos an der Oberfläche des Gehäuses 26 an und ist mit dieser verklebt. Die Kapselung 28 besitzt solche Abmessungen, daß in Ausbreitungsrichtung der genannten Anteile der Stoßwellen gemessen das eingeschlossene Luftvolumen eine Schichtdicke besitzt, die wenigstens gleich einer Wellenlänge einer mittels der Stoßwellenquelle 2 erzeugten, sich in dem Luftvolumen ausbreitenden Stoßwelle ist. Da es sich bei Luft um einen Stoff handelt, dessen akustische Impedanz von der des als akustisches Ausbreitungsmedium für die Stoßwellen vorgesehenen Wassers, dessen akustische Impedanz etwa gleich der von Körpergewebe ist, erheblich abweicht - sie ist um mehrere Zehnerpotenzen geringer als die von Wasser -, erfolgt an den Grenzflächen des Luftvolumens eine nahezu vollständige Reflexion der auf das Luftvolumen auftreffenden Anteile der Stoßwelle. Für die nicht reflektierten Anteile der Stoßwelle stellt das Luftvolumen, dessen Schichtdicke quer zur Mittelachse der Stoßwellenquelle 2 gemessen einige Millimeter beträgt, eine wirksame Schalldämmung dar, da bei einer angenommenen Frequenz der Grundschwingung der mittels der Stoßwellenquelle erzeugten Stoßwellen von 0,5 MHz die Wellenlänge der Grundschwingung bei einer angenommenen Schallausbreitungsgeschwindigkeit von 340 m/s geringer als 1 mm ist.

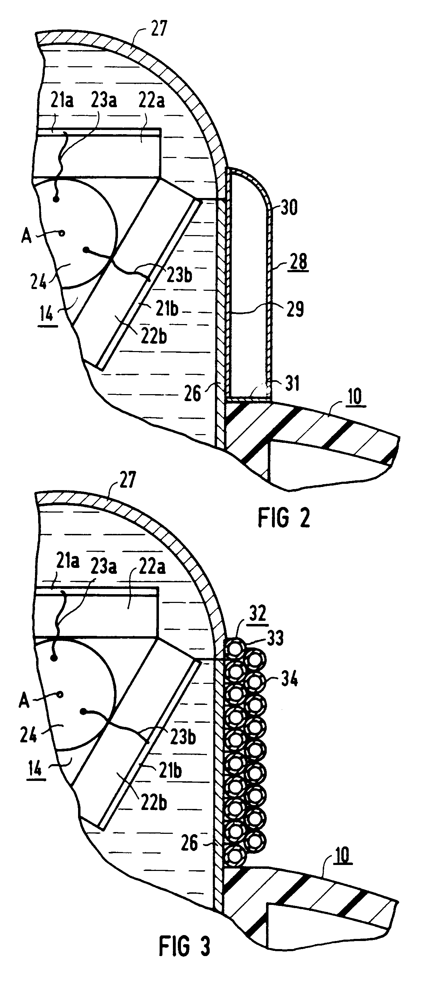

Im Falle der Fig. 1 ist das Luftvolumen, so wie dies aus der Fig. 2 näher ersichtlich ist, in der aus Messingblech gebildeten Kapselung 28 eingeschlossen. Diese besitzt eine zylinderrohrförmige Innenwand 29, die von einer sich an ihrem einen Ende verjüngenden Außenwand 30 umgeben ist. An ihrem einen Ende ist die Innenwand 29 mit dem entsprechenden Ende der Außenwand 30 z.B. durch Löten verbunden. An ihren anderen Enden sind Innen-und Außenwand 29, 30 mit einem ringförmigen Boden 31 z.B. ebenfalls durch Löten verbunden, so daß Innenwand 29, Außenwand 30 und Boden 31 der Kapselung 28 das innerhalb der Kapselung 28 befindliche Luftvolumen hermetisch dicht umschließen.

Gemäß einer in Fig. 3 dargestellten Variante ist das Luftvolumen in einem an seinen Enden dicht verschlossenen Kunststoff-Schlauch 32 eingeschlossen, der eine erste Lage 33, die in mehreren Windungen um das Gehäuse 26 gewickelt ist und in nicht dargestellter Weise mittels eines geeigneten Klebstoffes mit diesem verbunden ist, und eine zweite Lage 34 aufweist, die versetzt auf die erste Lage 33 gewickelt und mit dieser ebenfalls in nicht dargestellter Weise mittels des Klebstoffes verbunden ist. Da insbesondere bei der Verwendung einer entgasten Flüssigkeit als Ausbreitungsmedium nach und nach Luft durch das Kunststoffmaterial des Schlauches 32 in die Flüssigkeit diffundiert, sollte der Schlauch 32 eine ausreichend steife Wand besitzen, um zu vermeiden, daß er unter dem statischen Druck der Flüssigkeit plattgedrückt wird und das im Schlauch 32 eingeschlossene Luftvolumen nicht mehr in der beabsichtigten Schichtdicke vorliegt.

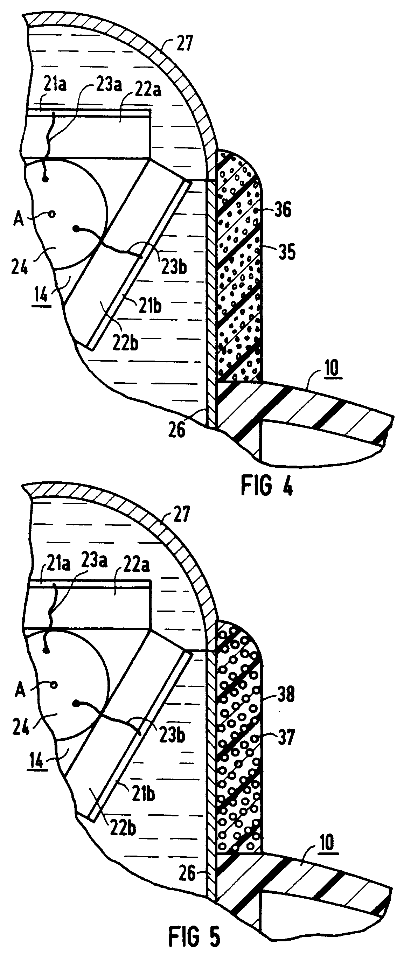

Das Luftvolumen kann auch in der in Fig. 4 dargestellten Weise in den Poren, eine davon trägt das Bezugszeichen 36, eines geschlossenporigen Schaumkörpers 35 eingeschlossen sein. Als Werkstoff kommt für den Schaumkörper 35 Polyurethanschaum in Frage. Der Schaumkörper 35 ist mittels eines geeigneten Klebstoffes in nicht dargestellter Weise mit dem Gehäuse 26 verklebt.

Eine weitere Möglichkeit, das Luftvolumen einzuschließen, ist in der Fig. 5 dargestellt. Hier befindet sich das Luftvolumen in einer Vielzahl von sehr kleinen Hohlkörpern, einer davon ist mit 37 bezeichnet, deren Größe ebenso wie die Größe der Poren 36 im Falle der Fig. 4 übertrieben dargestellt ist. Die Hohlkörper sind in einer Vergußmasse 38 eingebettet. Als Hohlkörper können hohle Glas- oder Kunststoffkügelchen verwendet werden. Als Vergußmasse 38, die zugleich dazu dient, die Hohlkörper 37 relativ zu dem Gehäuse 26 zu fixieren, eignet sich beispielsweise Silikon-Kautschuk.

Anstelle von Luft kann dem Ultraschall-Applikator 15 bzw. dessen Gehäuse 26 auch ein anderer gasförmiger, flüssiger oder fester Stoff vorgelagert sein, sofern dessen akustische Impedanz erheblich von der des akustischen Ausbreitungsmediums, bei dem es sich nicht notwendigerweise um Wasser handeln muß, abweicht. Aus den erläuterten Gründen kommen bevorzugt solche Stoffe in Frage, in denen die Schallausbreitungsgeschwindigkeit möglichst niedrig ist, da dann die zur Schalldämmung der nicht reflektierten Anteile der Stoßwellen erforderliche Schichtdicke des Stoffes entsprechend gering gehalten werden kann.

Im Falle erfindungsgemäßer Stoßwellengeneratoren können die zur Aufnahme des Luftvolumens bzw. des Stoffes dienenden Mittel 28, 32, 35, 37 sowohl mit dem eigentlichen Stoßwellengenerator als auch, so wie dies im Falle der Ausführungsbeispiele beschrieben ist, mit dem Ultraschall-Applikator 15 verbunden sein. Im Falle erfindungsgemäßer Ultraschall-Applikatoren versteht es sich, daß die genannten Mittel mit dem Ultraschall-Applikator verbunden oder wenigstens verbindbar sind.

Bei dem Ausführungsbeispiel nach Fig. 1 ist zur Fokussierung der Stoßwellen eine als Flüssigkeitslinse ausgebildete Sammellinse 10 vorgesehen. An deren Stelle kann eine als Feststofflinse ausgebildete Sammellinse treten.

Im Falle der Ausführungsbeispiele ist der Ultraschall-Applikator 15 jeweils als mechanischer Sektor-Scanner mit mehreren an einem Rotor angebrachten Ultraschall-Transducern 21a, 21b, 21c ausgeführt. Es kann aber auch ein mechanischer Sektor-Scanner mit nur einem eine oszillierende Bewegung ausführenden Ultraschall-Transducer vorgesehen sein. Außerdem besteht die Möglichkeit, einen Ultraschall-Applikator mit feststehendem Ultraschall-Transducer zu verwenden. In diesem Falle besteht bei Ausbildung des Ultraschall-Transducers als Phased Array oder Linear Array die Möglichkeit, auf elektronischem Wege einen Scan durchzuführen.

## Patentansprüche

1. Stoßwellengenerator zur Erzeugung von Stoßwellen in einem akustischen Ausbreitungsmedium mit einem Ultraschall-Applikator (15), mittels dessen für Ortungszwecke Ultraschallwellen in das akustische Ausbreitungsmedium einleitbar sind und der wenigstens einen Ultraschall-Transducer (21a, 21b, 21c) aufweist, der in einem Gehäuse (26) aufgenommen ist, **dadurch gekennzeichnet,** daß das Gehäuse (26) ein Schallaustrittsfenster (27) zum Austritt für die mittels des Ultraschall-Transducers erzeugten Ultraschall-Wellen aufweist, und daß wenigstens dem im Ausbreitungsweg der Stoßwellen befindlichen, an das Schallaustrittsfenster (27) im wesentlichen angrenzenden und dieses umgebenden Bereich des Ultraschall-Applikators (15) ein einziger Stoff vorgelagert ist, dessen akustische Impedanz erheblich von der des akustischen Ausbreitungsmediums abweicht, wobei die Schallausbreitungsgeschwindigkeit in dem Stoff erheblich geringer als in dem akustischen Ausbreitungsmedium ist.

2. Stoßwellengenerator nach Anspruch 1, **dadurch gekennzeichnet,** daß der Stoff in einer Schichtdicke vorhanden ist, die wenigstens gleich einer Wellenlänge der Grundschwingung einer sich in dem Stoff ausbreitenden Stoßwelle ist.

3. Stoßwellengenerator nach Anspruch 1 oder 2, **dadurch gekennzeichnet,** daß als Stoff ein gasförmiges Medium, insbesondere Luft, vorgesehen ist.

4. Stoßwellengenerator nach einem der Ansprüche 1 bis 3, **dadurch gekennzeichnet,** daß der Stoff entweder in den Poren (36) eines geschlossenporigen Schaumes (35) oder in einem Schlauch (32), welcher mehrere Windungen aufweisen kann, oder in in einem Trägermaterial (38) eingebetteten Hohlkörpern (37) oder in einer einen Hohlraum umschließenden Kapselung (28) eingeschlossen ist.

5. Medizinischer Ultraschall-Applikator (15) zur Verwendung in einem von akustischen Stoßwellen durchlaufenen Ausbreitungsmedium, welcher ein Gehäuse (26) und wenigstens einen innerhalb des Gehäuses (26) angeordneten Ultraschall-Transducer (21a, 21b, 21c) aufweist, der in einem Gehäuse (26) aufgenommen ist, **dadurch gekennzeichnet,** daß das Gehäuse (26) ein Schallaustrittsfenster (27) zum Austritt der mittels des Ultraschall-Transducers (21a, 21b, 21c) erzeugten Ultraschallwellen aufweist, und daß wenigstens dem an das Schallaustrittsfenster (27) im wesentlichen angrenzenden und dieses umgebenden Bereich des Gehäuses (26) ein einziger Stoff vorgelagert ist, dessen akustische Impedanz erheblich von der des Ausbreitungsmediums abweicht, wobei die Schallausbreitungsgeschwindigkeit in dem Stoff erheblich gringer als in dem Ausbreitungsmedium ist.

6. Ultraschall-Applikator nach Anspruch 5, **dadurch gekennzeichnet,** daß der Stoff in einer Schichtdicke vorhanden ist, die wenigstens gleich einer Wellenlänge der Grundschwingung einer sich in dem Stoff ausbreitenden Stoßwelle ist.

## Claims

1. Shockwave generator for the generation of shockwaves in an acoustic propagation medium with an ultrasonic applicator (15), by means of which for locating purposes ultrasonic waves can be conducted into the acoustic propagation medium and which has at least one ultrasonic transducer (21a, 21b, 21c), which is accommodated in a housing (26), characterized in that the housing (26) has a sound outlet window (27) for the issue of the ultrasonic waves generated by means of the ultrasonic transducer, and in that a single substance is introduced to at least the region of the ultrasonic applicator (15) which is located in the propagation path of the shockwaves, substantially adjoins the sound outlet window (27) and surrounds it, the acoustic impedance of which substance differs considerably from that of the acoustic propagation medium, whereby the sound propagation speed in the substance is considerably less than in the acoustic propagation medium.

2. Shockwave generator according to claim 1, characterized in that the substance is present in a layer thickness which is at least equal to a wavelength of the fundamental oscillation of a shockwave propagating in the substance.

3. Shockwave generator according to claim 1 or 2, characterized in that a gaseous medium, in particular air, is provided as the substance.

4. Shockwave generator according to one of claims 1 to 3, characterized in that the substance is enclosed either in the pores (36) of a closed-pore foam (35) or in a tube (32), which can have several turns, or in hollow bodies (39) embedded in a carrier material (38) or in a casing (28) surrounding a cavity.

5. Medical ultrasonic applicator (15) for use in a propagation medium through which acoustic shockwaves pass, which has a housing (26) and at least one ultrasonic transducer (21a, 21b, 21c) arranged within the housing (26), which transducer is accommodated in a housing (26), characterized in that the housing (26) has a sound outlet window (27) for the issue of the ultrasonic waves generated by means of the ultrasonic transducer (21a, 21b, 21c) and in that a single substance is introduced to at least the region of the housing (26) substantially adjoining the sound outlet window (27) and surrounding it, the acoustic impedance of which substance differs considerably from that of the propagation medium, whereby the sound propagation speed in the substance is considerably less than in the propagation medium.

6. Ultrasonic applicator according to claim 5, characterized in that the substance is present in a layer thickness which is at least equal to a wavelength of the fundamental oscillation of a shockwave propagating in the substance.

## Revendications

1. Générateur d'ondes de choc destiné à produire des ondes de choc dans un milieu de propagation d'ultrasons, comportant un applicateur d'ultrasons (15), au moyen duquel des ondes ultrasonores peuvent être introduites dans le milieu de propagation des ultrasons, à des fins de localisation, et qui comporte au moins un transducteur à ultrasons (21a,21b, 21c), qui est logé dans un boîtier (26), caractérisé par le fait que le boîtier (26) comporte une fenêtre de sortie des ultrasons (27) destinée à la sortie des ondes ultrasonores produites au moyen du transducteur à ultrasons, et qu'au moins en avant de la zone de l'applicateur à ultrasons (15), qui est dans le trajet de propagation des ondes de choc, qui jouxte sensiblement la fenêtre de sortie des ultrasons (27) et qui entoure cette dernière, est disposé un seul matériau, dont l'impédance acoustique diffère fortement de celle du milieu de propagation des ultrasons, la vitesse de propagation du son dans le matériau étant nettement plus petite que dans le milieu de propagation des ultrasons.

2. Générateur d'ondes de choc suivant la revendication 1, caractérisé par le fait que le matériau est présent dans une couche ayant une épaisseur au moins égale à une longueur d'onde de l'oscillation fondamentale d'une onde de choc se propageant dans le matériau.

3. Générateur d'ondes de choc suivant la revendication 1 ou 2, caractérisé par le fait que l'on prévoit comme matériau un milieu gazeux, notamment de l'air.

4. Générateur d'ondes de choc suivant l'une des revendications 1 à 3, caractérisé par le fait que le matériau est inséré soit dans les pores (36) d'une mousse (35) à pores fermés, soit dans un tuyau (32), qui peut comporter plusieurs spires, soit dans des corps creux (37), qui sont insérés dans un matériau de support (38), soit dans un capotage (28) enfermant une cavité.

5. Applicateur médical à ultrasons (15), qui est destiné à être utilisé dans un milieu de propagation traversé par les ondes de choc ultrasonores et qui comporte un boîtier (26) et au moins un transducteur à ultrasons (21a,21b,21c), qui est disposé à l'intérieur du boîtier (26), caractérisé par le fait que le boîtier (26) comporte une fenêtre de sortie des ultrasons (27) destinée à la sortie des ondes ultrasonores produites au moyen du transducteur à ultrasons (21a,21b,21c) et qu'au moins en avant de la zone du boîtier (26), qui jouxte sensiblement la fenêtre de sortie des ultrasons (27) et qui entoure cette dernière, est disposé un seul matériau, dont l'impédance acoustique diffère fortement de celle du milieu de propagation, la vitesse de propagation du son dans le matériau étant nettement plus petite dans le matériau que dans le milieu de propagation des ultrasons.

6. Applicateur à ultrasons suivant la revendication 5, caractérisé par le fait que le matériau est présent dans une couche ayant une épaisseur au moins égale à une longueur d'onde de l'oscillation fondamentale d'une onde de choc se propageant dans le matériau.
